# EUROPÄISCHE PATENTSCHRIFT

(11) **EP 1 105 511 B1**
(45) Veröffentlichungstag und Bekanntmachung des Hinweises auf die Patenterteilung: **31.10.2007**
(21) Anmeldenummer: 99942852.7
(22) Anmeldetag: 11.08.1999
(51) Int. Cl.: C12N 15/82, C07K 14/415, C12N 5/10, A01H 5/00

(54) **TRANSGENE PFLANZEN UND PFLANZENZELLEN MIT VERMINDERTER EXPRESSION VON INVERTASEINHIBITOREN**
TRANSGENIC PLANTS AND PLANT CELLS COMPRISING A REDUCED EXPRESSION OF INVERTASE INHIBITORS
PLANTES TRANSGENIQUES ET CELLULES VEGETALES PRESENTANT UNE EXPRESSION REDUITE DES INHIBITEURS D'INVERTASE

(30) Priorität: 12.08.1998 DE 19836405
(43) Veröffentlichungstag der Anmeldung: 13.06.2001
(73) Patentinhaber: Rausch, Thomas, 69120 Heidelberg (DE)
(72) Erfinder: Rausch, Thomas, 69120 Heidelberg (DE)
(74) Vertreter: Schrell, Andreas
(86) Internationale Anmeldenummer: PCT/EP1999/005890
(87) Internationale Veröffentlichungsnummer: WO 2000/009719

(56) Entgegenhaltungen:
- EP-A- 0 442 592
- WO-A-97/07221
- WO-A-98/04722
- GREINER, S., ET AL. : "cloning of a tobacco apoplasmic invertase inhibitor" PLANT PHYSIOLOGY, Bd. 116, Februar 1998 (1998-02), Seiten 733-742, XP002125613 in der Anmeldung erwähnt
- KRAUSGRILL, S., ET AL. : "in transformed tobacco cells the apoplasmic invertase inhibtor operates as a regulatory switch of cell wall invertase" THE PLANT JOURNAL, Bd. 13, Nr. 2, Januar 1998 (1998-01), Seiten 275-280, XP002125614
- KRAUSGRILL S ET AL: "REGULATION OF CELL WALL INVERTASE BY A PROTEINACEOUS INHIBITOR" JOURNAL OF EXPERIMENTAL BOTANY,GB,OXFORD UNIVERSITY PRESS, Bd. 47, Seite 1193-1198 XP002050469 ISSN: 0022-0957
- SANDER A ET AL: "SUCROSE PROTECTS CELL WALL INVERTASE BUT NOT VACUOLAR INVERTASE AGAINST PROTEINACEOUS INHIBITORS" FEBS LETTERS,NL,ELSEVIER SCIENCE PUBLISHERS, AMSTERDAM, Bd. 385, Nr. 3, Seite 171-175 XP002049864 ISSN: 0014-5793

## Beschreibung

Die vorliegende Erfindung betrifft transgene Pflanzenzellen und Pflanzen, Verfahren für deren Bereitstellung sowie die Verwendung von Invertaseinhibitor-cDNA-Sequenzen in antisense- oder sense-Orientierung zur Herstellung solcher Pflanzen.

Die Verbesserung von Qualität und/oder Quantität pflanzlicher Reservestoffe in Samen dicotyler und monocotyler landwirtschaftlicher Nutzpflanzen stellt ein wichtiges Ziel biotechnologischer Forschung dar. In der Regel wurden bisher Strategien entwickelt, die auf der Einführung bestimmter Gene basieren, deren Genprodukte Enzyme darstellen, die an der Synthese des Reservestoffes selbst (z.B. ADP-Glucosepyrophosphorylase) beteiligt sind. Desweiteren wurden aber auch Verfahren beschreiben, in denen durch veränderte Expression von heterologen und damit deregulierten Invertasen bzw. Glucokinasen im Cytosol eine erhöhte Glycolyserate erreicht wird (DE-A1-195 29 696). In letzterer Variante führt die erhöhte Spaltung von Saccharose durch eine deregulierte pilzliche Invertase in Kombination mit einer deregulierten bakteriellen Glucokinase zu einer verstärkten Glycolyserate. Dieser Ansatz beruht auf der Annahme, daß auf Grund der erhöhten Konzentrationen an Intermediaten der Glycolyse die Synthese von Speicherölen in Samen stimuliert wird, da die Metabolisierung des primären Photoassimilats Saccharose zu phosphorylierten Hexosen bzw. den Fettsäurevorstufen Pyruvat und Acetyl-CoA gefördert wird.

Die DE-A1-195 29 696 beschreibt demgemäß die Einführung eines artfremden, z.B. pilzlichen Gens zur Expression der Invertase. Dieses pilzliche Invertase-Enzym, das artfremd ist und deshalb keiner Regulation unterliegt, wird aufgrund der Zuführung des fremden Gens unter der Regulation eines geeigneten Promotors verstärkt gebildet, wodurch der von der Invertase katalysierte Abbau der Saccharose in Glucose und Fructose beschleunigt erfolgt. Die mit höherer Geschwindigkeit erfolgende Bildung der Glucose soll letztendlich eine beschleunigte Produktion von pflanzlichen Speicherstoffen bewirken. Dieses Verfahren beruht auf einem Eingriff in den Metabolismus in der Zelle des Samenspeichergewebes, wobei der Assimilattransfer zwischen maternalen und Samenparenchym nur indirekt beeinflußt wird.

Die Bedeutung von Zellwand-Invertasen für die Entwicklung stärke- und proteinreicher Samen ist bekannt. So wird z.B. die Stärkeakkumulation in Maissamen bei verminderter Expression einer Zellwand-Invertase durch Störung des Assimilattransfers zwischen Pedicel und Endosperm gestört. Für verschiedene Pflanzenspezies sind blütenspezifische Zellwand-Invertase-Isoformen bekannt. Für Nicotiana tabacum konnte gezeigt werden, daß ein apoplastischer Invertaseinhibitor besonders im Ovar und den Staubblättern stark exprimiert wird. Greiner et al. (Plant Physiol.(1998), 733-742) offenbart die Aminosäure- und cDNA-Sequenz des genannten Invertaseinhibitors sowie dessen in vitro-Funktionsnachweis mittels heterolog exprimiertem Inhibitorprotein. Hingegen wurde eine in vivo-Hemmung noch nicht gezeigt. Bekannt ist es überdies, daß in verschiedenen Geweben und zu verschiedenen Zeitpunkten der pflanzlichen Entwicklung unterschiedliche Isoformen von Zellwandinvertasen und Invertaseinhibitoren vorliegen.

Eine gezielte Zuordnung der Aktivitäten und deren möglichen Zusammenwirken dieser zeit- und gewebespezifisch auftretenden beiden Proteine ist bisher nicht möglich. Untersuchungen zur in-vivo-Situation hinsichtlich der Regulation von Zellwand-Invertasen durch Invertaseinhibitoren sind nicht bekannt. Ebensowenig war bekannt, ob und wenn ja welche Isoformen der Zellwandinvertasen während der Samenentwicklung einer endogenen Regulation durch Invertaseinhibitoren unterliegen und wenn ja, welche Isoformen der Invertaseinhibitoren. Die gezielte Nutzung dieser Proteine für die Herstellung vorteilhafter Pflanzen ist daher bisher nicht möglich.

Der Erfindung liegt daher das technische Problem zugrunde, transgene Pflanzenzellen, Pflanzen und diese herstellende Verfahren bereitzustellen, wobei die Pflanzen sich durch die Bildung von Samen auszeichnen, die gegenüber Samen von nicht transformierten Pflanzen eine größere Menge pflanzlicher Speicherstoffe wie Kohlenhydrate, Fette oder Proteine aufweisen, ohne daß dabei endogene oder exogene Proteine überexprimiert werden und der Phänotyp der Pflanze sowie deren Entwicklung beeinträchtigt werden.

Das der vorliegenden Erfindung zugrundeliegende technische Problem wird durch ein Verfahren zur Herstellung einer transgenen Pflanze mit einer deregulierten und die Samenentwicklung fördernden Invertaseaktivität bereitgestellt, wobei das Verfahren vorsieht, aus einer cDNA-Bank einer Zellsuspensionskultur oder von Blüten mit jungen Samenanlagen aus einer Pflanze eine Nukleotidsequenz eines Invertaseinhibitors zu gewinnen oder davon abzuleiten, eine Pflanzenzelle einer Pflanze derselben Art oder Sorte mit einem DNA-Konstrukt, enthaltend die funktionell mit mindestens einer regulatorischen Einheit verbundene Nucleotidsequenz eines Invertaseinhibitors zu transformieren, zu kultivieren und zu einer Pflanze zu regenerieren, deren Samen im Vergleich zu nicht mit einem solchen DNA-Konstrukt transformierten Pflanzen eine größere Menge an Speicherstoffen wie Kohlenhydrat, Fett oder Protein bildet.

Die Erfindung sieht insbesondere vor, transgene Pflanzen mit einer veränderten Expression eines, vorzugsweise apoplastischen, Invertaseinhibitors herzustellen, wobei sich die Pflanzen dadurch auszeichnen, das die Expression von Invertaseinhibitorproteinen während der Samenentwicklung reduziert oder ganz eliminiert ist. Das Verfahren läßt sich vorteilhafter Weise auf die unterschiedlichsten dicotylen oder monocotylen Nutzpflanzen anwenden z.B. Raps, Sonnenblumen, Erdnuß, Ölpalme, Sojabohne, Calendula officinalis, Coriandrum sativum, Crambe abyssinica, Cuphea ssp., Dimorphotheca pluvialis, Euphorbia lagascae, Euphorbia lathyris, Lesquerella grandiflora, Limnanthes alba, Linum usitatissimum, Lunaria annua, Lunaria biennis, Oenothera ssp., Ricinus communis, Simmondsia chinensis als Pflanzen mit Fett speichernden Samen; Mais, Reis, Weizen, Gerste, Hafer, Roggen als Pflanzen mit Stärke speichernden Samen; und beispielsweise Sojabohne oder Erbse als Pflanzen mit Protein speichernden Samen.

Die Erfindung sieht also vor, eine Pflanzenzelle mit einer unter Kontrolle mindestens einer regulatorischen Einheit stehenden Nucleotidsequenz eines Invertaseinhibitorgens zu transformieren, wobei die Nucleotidsequenz des Invertaseinhibitorgens zur Elimination oder Reduzierung der Aktivität eines zelleigenen, endogenen Invertaseinhibitors befähigt ist. In bevorzugter Ausführungsform kann die Elimination der Aktivität eines endogenen Invertaseinhibitors in der Zelle dadurch erreicht werden, daß die Nucleotidsequenz eines Invertaseinhibitors in einem antisense-DNA-Konstrukt eingesetzt wird, d.h. ein Konstrukt, in dem eine Nucleotidsequenz des Invertaseinhibitorgens in antisense-Orientierung zu einem Promotor vorhanden ist. Durch die Expression, d.h. im vorliegenden Kontext die Transcription des antisense-Konstrukts wird die Aktivität des zelleigenen Invertaseinhibitorgens gehemmt oder reduziert, so daß die so deregulierte Invertase zu einer erhöhten Speicherstoffakkumulation im Samen führt.

Im Zusammenhang mit der vorliegenden Erfindung wird unter einem antisense-Konstrukt ein DNA-Konstrukt verstanden, welches eine in antisense-Orientierung zu einem Promotor funktionell verbundene Nucleotidsequenz eines Invertaseinhibitors aufweist, wobei diese Nucleotidsequenz entweder die full-length-cDNA des Invertaseinhibitors, eine davon abgeleitete Sequenz oder ein Fragment, allelische Variante oder Derivat davon ist.

Im Zusammenhang mit der vorliegenden Erfindung wird unter einer von einer cDNA abgeleiteten Sequenz eine mit dieser cDNA-Sequenz hybridisierende künstliche oder natürliche Nucleotidsequenz verstanden, also eine Nucleotidsequenz, die unter den in Sambrook et al. (Molecular Cloning, a laboratory manual, 2nd edition (1989), Cold Spring Harbor Laboratory Press) beschriebenen Bedingungen mit der cDNA-Sequenz des Invertaseinhibitors hybridisiert, vorzugsweise unter stringenten Bedingungen. Erfindungsgemäß weisen hybridisiernde Sequenzen eine Sequenzidentität von 60, 70, 80, 90, 95 oder 97% besonders bevorzugt 99%, zu der cDNA-Sequenz eines Invertaseinhibitorgens auf. Sofern erfindungsgemäß Fragmente einer cDNA-Sequenz eines Invertaseinhibitors verwendet werden, weisen die Fragmente zumindest eine Länge und Sequenzähnlichkeit auf, die ausreicht, durch Hybridisierung zu Wildtyp-Transcript die Translation einer endogen gebildeten Invertaseinhibitor mRNA zu inhibieren, beispielsweise eine Länge von wenigen hundert Basenpaaren. Selbstverständlich kann auch vorgesehen sein, daß die antisense-Konstrukte Nucleotidsequenzen eines Invertaseinhibitorgens aufweisen oder aus diesen bestehen, die transcribiert, aber nicht translatiert sind, d.h. nicht translatierte Regionen, sogenannte UTR's.

Im Zusammenhang mit der vorliegenden Erfindung sind unter DNA-Konstrukten, die die Elimination oder Reduktion der Aktivität eines endogenen Invertaseinhibitorgens bewirken können, auch DNA-Konstrukte zu verstehen, die eine wie vorstehend definierte Nucleotidsequenz eines Invertaseinhibitors oder eine davon abgeleitete Sequenz aufweisen, die funktionell in sense-Orientierung mit mindestens einer regulatorischen Einheit, z.B. einem Promotor, verbunden sind. Mit derartigen Konstrukten kann durch Cosupression die Bildung endogener Invertaseinhibitoren verhindert werden, z.B. dadurch, daß eine Vielzahl von sense-Kopien der Nucleotidsequenz eines Invertaseinhibitors in dem Genom der transformierten Zelle vorhanden sind und die Expression endogener Invertaseinhibitoren eliminieren.

Die erfindungsgemäßen Konstrukte sind vorzugsweise in einem Vektor angeordnet, z.B. einem Plasmid, Virus, Cosmid, Bakteriophagen oder einem sonstigen in der Gentechnik üblichen Vektor.

Erfindungsgemäß kann vorgesehen sein, die einzusetzende Nucleotidsequenz eines Invertaseinhibitors nicht nur funktionell mit einem 5'-wärts gelegenen Promotor zu verbinden, sondern vorteilhafter auch 3'-wärts der Nucleotidsequenz ein Transcriptionsterminationssignal, z.B. aus dem NOS-Gen von Agrobakterium tumefaciens, einzusetzten. Selbstverständlich ist es auch möglich, in dem Vektor weitere funktionelle Einheiten vorzusehen, wie T-DNA border-Sequenzen oder die Vektoren stabilisierende Elemente.

Die Erfindung stellt in bevorzugterweise also Pflanzen bereit, die in ihren Samen eine im Vergleich zu nicht transformierten Pflanzen erhöhte Speicherstoffmenge aufweisen, wobei eine im Vergleich erhöhte Speicherstoffmenge bedeutet, daß die durchschnittliche Menge des untersuchten Speicherstoffs in Samen einer Gesamtheit von transformierten Pflanzen um vorzugsweise 5, bevorzugt 10, 20, 30 40, 50 besonders bevorzugt 90, 100, 200 oder 300% höher ist als die durchschnittliche Menge des in Frage stehenden Speicherstoffes im Samen von einer Gesamtheit nicht transformierter Pflanzen.

Die Erfindung betrifft daher die überraschende Lehre, daß mittels einer Nucleotidsequenz eines Invertaseinhibitorgens, insbesondere einer cDNA eines Invertaseinhibitorgens, Pflanzen hergestellt werden können, die in-vivo eine erhöhte Speicherstoffakkumulation im Samen aufweisen, ohne daß die Entwicklung der Pflanze in anderer Hinsicht verändert oder beeinträchtigt wird. Die Erfindung beruht dabei unter anderem auf der überraschenden Tatsache, daß die endogenen Invertasen bei der Samenentwicklung einer endogenen Regulation durch Invertaseinhibitoren unterliegen.

Die Erfindung betrifft daher auch die Verwendung einer funktionell mit mindestens einer regulatorischen Einheit verbundenen Nucleotidsequenz eines Invertaseinhibitorgens zur Transformation und Herstellung von Pflanzen, die eine modifizierte Samenentwicklung aufweisen, insbesondere Samen bilden, die eine erhöhte Speicherstoffmenge gegenüber Samen nicht transformierter Pflanzen aufweisen.

Die Erfindung betrifft insbesondere die vorgenannte Verwendung einer Nucleotidsequenz eines Invertaseinhibitorgens, wobei diese aus einer cDNA-Bank einer Zellsuspensionskultur oder aus Blüten mit jungen Samenanlagen der Pflanzenart oder gegebenfalls -sorte gewonnen oder davon abgeleitet wurde, die erfindungsgemäß mit dem mittels dieser Nucleotidsequenz hergestellten DNA-Konstrukt der vorliegenden Erfindung zu transformieren ist. Die für die Transformation eingesetzte Nucleotidsequenz ist also die Nucleotidsequenz oder ist davon abgeleitet, die die in der Zellsuspensionskultur oder in den Blüten mit jungen Samenanlagen vorherrschende Isoform des Invertaseinhibitors codiert.

Im Zusammenhang mit der vorliegenden Erfindung sind Blüten mit jungen Samenanlagen Blüten mit unreifen Samenanlagen, das heißt nach Bestäubung aber vor Beginn der Dormanz.

Die Lösung des technischen Problems ist erfindungsgemäß auch eine transgene Pflanzenzelle, bei der im Vergleich zu nicht transformierten Pflanzenzellen aufgrund verminderter Expression von Invertaseinhibitoren eine erhöhte Aktivität der Zellwand-Invertase vorliegt, wobei diese verminderte Expression durch Einführung einer der gleichen Pflanzenarten entsprechenden (homologen) und unter Regulation eines Promotors stehenden Invertaseinhibitor-cDNA in antisense-Orientierung ausgelöst wird.

Erfindungsgemäß wird auch ein Verfahren zur Gewinnung von Invertaseinhibitor-cDNA-Sequenzen in sense- oder antisense-Orientierung bereitgestellt, wobei dieses - unabhängig von der jeweiligen Spezies - folgende Schritte enthält:
a) Gewinnung einer Inhibitorproteinfraktion aus der Zellwandprotein-Fraktion einer entsprechenden Zellsuspensionskultur
b) Gewinnung von entsprechenden Peptidsequenzen nach Spaltung und Reinigung der entstandenen Peptide
c) Clonierung von zunächst partieller und in der Folge Vollängen-cDNA für das Invertaseinhibitorprotein aus einer cDNA-Bank
d) Clonierung der Invertaseinhibitor-cDNA in sense oder antisense-Orientierung in einen Vektor, z.B. einen binären Vektor
e) Transformation der Pflanzenspezies mit dem sense- oder antisense-Genkonstrukt.

Der Assimilattransfer zwischen maternalem Gewebe und Samengewebe ist der geschwindigkeitsbestimmende Schritt bei der Bildung der pflanzlichen Speicherstoffe im Samen. Wird dieser Schritt durch die Erhöhung der Aktivität der in der Übergangszone exprimierten Zellwand-Invertase beschleunigt, so kommt es in Folge des verstärkten Assimilattransfers (die erhöhte Aktivität der Zellwand-Invertase bewirkt eine Steigerung des Assimilattransfers zwischen maternalem und Samengewebe) zu einer erhöhten Akkumulation des hauptspeicherstoffs der jeweiligen Pflanzenspezies (Stärke, Fett,Protein).

Die Erfindung betrifft auch ein Verfahren zur Herstellung der vorgenannten Pflanzen, wobei in einem ersten Schritt aus einer Zellsuspensionskultur oder aus Blüten mit jungen Samenanlagen eine Inhibitorproteinfraktion, insbesondere die vorherrschende Inhibitorproteinfraktion, aus einer Zellwandproteinfraktion gewonnen wird, anschließend in einem zweiten Schritt die Inhibitorproteinfraktion gereinigt ggf. gespalten und zumindest N-terminal sequenziert wird, so daß aus der so gewonnenen Aminosäuresequenz eine Nucleotidsequenz abgeleitet werden kann, im Rahmen eines dritten Schritts mittels beispielsweise Primern, partielle oder full-length cDNA für das Invertaseinhibitorprotein auseiner cDNA-Bank einer Zellsuspensionskultur oder von Blüten mit jungen Samenanlagen der gleichen, vorgenannten Pflanze bzw. Sorte cloniert wird, anschließend in einem vierten Schritt die gewonnene cDNA in sense- oder antisense-Orientierung in einen Vektor cloniert wird, um anschließend in einem fünften Schritt eine Pflanzenzelle gleicher Art oder Sorte mit dem so gewonnenen DNA-Konstrukt zu transformieren, wobei dies die Art oder Sorte ist, aus der die cDNA, und die Aminosäuresequenz für die cDNA-Isolierung gewonnen wurde.

Die Erfindung betrifft daher auch gemäß des vorliegenden Verfahrens hergestellte Pflanzen, Pflanzenteile wie Wurzel, Stengel, Blätter, Ernte- und Vermehrungsmaterial wie Früchte, Pollen, Samen, Samenschale, Embryo, Sämlinge, Zellkulturen, Kalli etc. Die Erfindung betrifft dabei jegliche Pflanzensorte oder Pflanzenart und weist demgemäß keinerlei Sorten- oder Artspezifität auf. Das erfindungsgemäße Verfahren stellt ein im wesentlichen technisches Verfahren dar, wobei in dessen Rahmen eine gezielte Zuordnung vom Ausgangsmaterial für die zu verwendenden Mittel, wie cDNA-Sequenzen, zu zu transformierender, Pflanze d.h Zielobjekt, gegeben wird.

Im Gegensatz zu allen bisherigen Verfahren beruht daher die hier beschriebene Erfindung auf der Regulation spezifischer, während der Samenentwicklung exprimierter Zellwand-Invertase-Isoformen. Die Invertaseinhibitor-cDNA codiert in bevorzugter Ausführungsform eine apoplastische Variante des Invertaseinhibitors. Durch Einführung einer einzigen autologen, d.h. aus dem zu transformierenden Organismus stammenden bzw. davon abgeleiteten cDNA-Sequenz pflanzlichen Ursprungs wird der für den Assimilattransfer entscheidende Schritt der Saccharosespaltung am natürlichen Wirkungsort beeinflußt. Die Beobachtung, daß die Einführung mindestens einer Sequenz, das heißt einer Invertaseinhibitor-cDNA, in sense- oder antisense-Orientierung unter der Steuerung z.B. des konstitutiven CaMV35S-Promotors, des Ubiquitin-Promotors oder Zein-Promotors aus Mais oder eines Promotors ähnlich hoher oder größerer Aktivität, z.B. auch eines gewebespezifischen Promotors, die gesamte vegetative Pflanzenentwicklung nicht beeinflußt, sondern nur während der Samenentwicklung zu einer spezifischen Deregulierung führt, zeigt die extrem hohe Spezifität des transgenen Eingriffs. Die Vorteile dieser direkten Deregulierung sind offensichtlich: 1) es genügt ein einziges Genkonstrukt, um eine signifikante Veränderung der Reservestoffakkumulation zu erreichen, 2) es werden keine artfremden Genprodukte gebildet, 3) der Eingriff im Metabolismus ist hochgradig spezifisch, 4) für Tabak wird exemplarisch gezeigt, daß die veränderte Expression eines apoplastischen Invertaseinhibitors zu drastischen Änderungen der Speicherölbildung führt.

Die Beeinflussung, insbesondere Erhöhung der Akkumulation der Samenspeicherstoffe durch eine gezielte Veränderung der Expression des Invertaseinhibitors, insbesondere Verminderung, beruht unter anderem auf folgenden Mechanismen:
a) Durch die Veränderung der Aktivitätsphase der Zellwand-Invertase im maternalen Gewebe wird die Effizienz der Nährstoffentladung beeinflußt, das heißt z.B. in Inhibitor-antisense-Transformanten, erhöht.
b) Für die Synthese von Reserveölen des Samens ist der oxidative Pentosephosphatzyklus von entscheidender Bedeutung. Die anhaltend erhöhte Bereitstellung von Glucose in z.B. Inhibitor-antisense-Transformanten fördert daher die Speicherölsynthese.
c) Durch die Veränderung des Verhältnisses von Hexosen zu Saccharose wird die Zellteilungsphase der Samenentwicklung beeinflußt. Durch eine Verlängerung der Aktivitätsphase der Zellwand-Invertase in z.B. Inhibitor-antisense-Transformanten wird z.B. die Zellzahl pro Samen erhöht.

Im Vergleich zur ebenso möglichen Überexpression der am Assimilattransfer beteiligten Zellwand-Invertase(n) hat der hier beschriebene indirekte Ansatz einer Ausschaltung der Invertaseinhibitoren noch weitere Vorteile. Die im Verlauf der Samenbildung exprimierten Zellwand-Invertasen werden bereits natürlicherweise stark exprimiert, das Ausmaß einer zusätzlichen Induktion durch Einsatz starker Promotoren ist daher begrenzt, wohingegen durch die erfindungsgemäße antisense-Ausschaltung des Inhibitors eine starke Erhöhung der Zellwand-Invertase(n)-Aktivität erreicht werden kann. Zwar könnte durch Expression einer heterologen, deregulierten, inhibitorinsensitiven Invertase mit Signalpeptid für die Zielsteuerung in den Zellwandraum ein ähnlicher Effekt erreicht werden, doch müssen in diesem Fall artfremde Proteine eingesetzt werden. Außerdem besteht bei einer Kombination der für diesen Ansatz notwendigen samenspezifischen Promotoren mit einer deregulierten Invertase die große Gefahr, daß eine zu hohe Expression zu unerwünschten Nebenwirkungen führt. Im Gegensatz hierzu wird beim hier beschriebenen Verfahren die maximale Aktivität der natürlicherweise vorkommenden Zellwand-Invertasen nie überschritten, es wird lediglich die Zeitspanne ihrer Aktivität während der Reservestoffakkumulation ausgedehnt. Aus diesen Gründen ist die indirekte Regulation der Zellwand-Invertasen über antisense-Expression von Invertaseinhibitoren oder im Rahmen der Cosuppressiontechnologie über sense-DNA-Konstrukte in jedem Falle der Einführung einer heterologen Invertase vorzuziehen und stellt eine bedeutsame technische Verbesserung dar.

Methoden zur Gewinnung einer homogenen Inhibitorproteinfraktion aus der apoplastischen Zellwandproteinfraktion einer Zellsuspensionskultur

Von der jeweiligen Pflanzenspezies wird eine Zellsuspensionskultur angelegt. Die Verfahren zur Gewinnung einer Zellkultur folgen Standardprotokollen der pflanzlichen Gewebekultur. In der Regel werden die Zellen unter sterilen Bedingungen in einem komplexen Nährmedium unter Zusatz von Saccharose (Kohlenstoffquelle) als Schüttelkultur angezogen. Unter diesen Anzuchtbedingungen exprimieren pflanzliche Zellen eine Zellwand-Invertase, die durch einen ebenfalls exprimierten Invertaseinhibitor reguliert wird.

Die Anreicherung und Reinigung des Invertaseinhibitors basiert auf dessen Bindung an die Zellwandinvertase. Zunächst wird eine Zellwandproteinfraktion durch Inkubation in 1 M NaCl, 1 mM PMSF bei 4°C unter Schütteln extrahiert. Hierbei werden in der Regel keine cytosolischen Proteine extrahiert. Die so gewonnene Zellwandproteinfraktion wird über Ammoniumsulfatfällung (80%) bzw. über Membranfiltration konzentriert. Durch anschließende Chromatographie an einer Concanavalin A-Säule wird eine Glycoproteinfraktion gewonnen, die die glycosylierte Zellwand-Invertase und den an diese gebundenen Invertaseinhibitor enthält. SDS-PAGE/Western blot-Analysen der so gewonnenen Zellwand-Invertase- und damit Invertaseinhibitor-angereicherten Fraktionen mit einem polyclonalen Antiserum gegen den Invertaseinhibitor aus Tabakzellen zeigen die Präsenz von Invertaseinhibitoren, in der Regel Protein von 15-25 kDa, an.

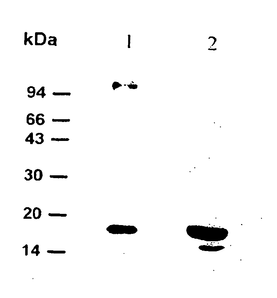

Die Figur 1 zeigt dazu den Nachweis von zum Tabak-Invertaseinhibitor homologen Invertaseinhibitoren anderer Pflanzenarten - eine Western Blot Analyse von aus Suspensionskulturen von Chenopodium rubrum (1) und Daucus carota (2) gewonnenen Zellwandprotein-Proben. Die Entwicklung wurde mit einem gegen den rekombinanten Tabak-Invertaseinhibitor gewonnenen Antiserum vorgenommen. Für beide Spezies wurden Invertaseinhibitor-Polypeptide von ca. 17 kDa detektiert.

Die weitere Reinigung der Komplexe aus Zellwand-Invertase und Invertaseinhibitor erfolgt über Ionenaustauscherchromatographie an einem Kationenaustauscher, z.B. Sulfopropylsephadex. Nach sequentieller Chromatographie, zunächst über einen pH-Gradienten (pH 8-12), danach über einen NaCl-Gradienten, wird eine stark angereicherte Präparation der Zellwand-Invertase gewonnen, wobei der Invertaseinhibitor im stabilen Komplex mit letzterer vorliegt. Die Peak-Fraktionen der letzten Ionenaustauscherreinigung werden über SDS-PAGE/Western blot-Analyse auf Zellwand-Invertase hin detektiert, z.B. mit einem Antiserum gegen die Karotten-Zellwand-Invertasen. Außerdem werden die Invertase-Aktivitäten aller Fraktionen im gekoppelten enzymatischen Test mit Hexokinase/Glucose-6-Phosphat-Dehydrogenase ermittelt. Die Fraktionen mit starkem Zellwand-Invertase-Immunosignal aber geringer Invertaseaktivität enthalten das in der Regel hochreine Inhibitorprotein.

Methoden zur Gewinnung von Peptidsequenzen der gereinigten Invertaseinhibitoren

Das Inhibitorprotein liegt nach dem oben beschriebenen Reinigungsprotokoll ausreichend rein vor, um nach Elektroblot auf eine PMDF-Membran direkt N-terminal ansequenziert zu werden. Nach Gewinnung von 100-500 µg Inhibitorprotein kann dieses ggf. erneut über SDS-PAGE gereinigt und anschließend direkt im Gel tryptisch verdaut werden. Die Trennung der entstehenden Peptide über reverse phase-HLPC und deren anschließende Sequenzierung über Edmann-Abbau entspricht Standardverfahren. Die Kombination von N-terminaler Ansequenzierung und der Sequenzierung der beim tryptischen Verdau erhaltenen Peptide führt zu ausreichender Sequenzinformation für eine auf dem RT-PCR-Verfahren basierende Clonierung.

Verfahren zur Clonierung von zunächst partiellen und in der Folge Vollängen-cDNAs für das jeweilige Invertaseinhibitorprotein aus einer cDNA-Bank

Ausgehend von den erhaltenen Peptidsequenzinformationen werden gemäß des genetischen Codes Primersequenzen abgeleitet. Für das optimale Primerdesign werden Standardalgorithmen benutzt. In einer anderen Ausführung werden Primer an Hand hochkonservierter Sequenzbereiche der bereits bekannten Invertaseinhibitorsequenzen aus Nicotiana tabacum, Lycopersicon esculentum, Arabidopsis thaliana und Citrus inshui entworfen.

Zunächst wird eine 1.Strang cDNA-Synthese nach Standardverfahren durchgeführt. Hierfür wird Gesamt-RNA aus einer Zellsuspensionskultur, bzw. in einer anderen Ausführung aus Blüten mit jungen Samenanlagen nach Standardverfahren extrahiert. Über RT-PCR wird dann zunächst eine partielle Invertaseinhibitor-cDNA amplifiziert. Das Amplifikat wird in einer Ausführung in den Blueskript-Vektor cloniert. Nach Sequenzierung und Bestätigung einer zu den bereits bekannten Invertaseinhibitoren homologen Sequenz wird diese partielle cDNA als Sonde für die Gewinnung von Vollängenclonen eingesetzt. Hierfür wird nach Standardverfahren entweder eine cDNA-Bank aus einer Zellsuspensionskultur, oder in einer anderen Ausführung, eine cDNA-Bank aus Blüten mit jungen Samenanlagen angelegt.

Clonierung der Invertaseinhibitor cDNA in sense- bzw. antisense-Orientierung in einen Vektor, z.B. einen binären Vektor

Die für jede Pflanzenart clonierte Invertaseinhibitor-cDNA wird in einer Ausführungsform in den binären Vektor BinAR (Bin19 Derivat)(Höfgen und Willmitzer, Plant Sci. 66 (1990), 221-230) cloniert. Sowohl für antisense- wie auch für sense-Konstrukte wird in einer Ausführungsform der CaMV35S-Promotor verwendet, doch können gegebenenfalls auch andere, z.B. gewebespezifische Promotoren verwendet werden.

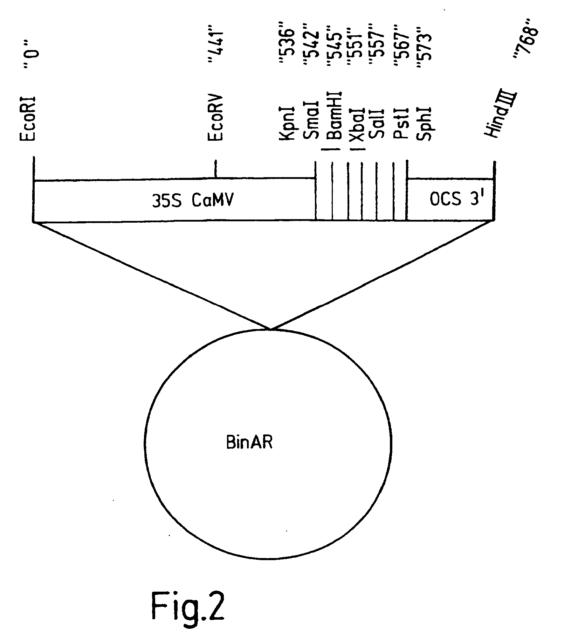

Figur 2 zeigt den binären Vektor (BinAR) zur Agrobakterium tumefaciens-vermittelten Transformation. Die codierenden Regionen der InvertaseinhibitorcDNAs werden in sense- oder antisense-Orientierung in die 'multiple cloning site' cloniert.

Transformation der jeweiligen Pflanzenspezies mit den sense- bzw. antisense-Genkonstrukten

Für die meisten dicotylen Nutzpflanzen werden Invertaseinhibitor-sense/antisense-Transformanten unter Einsatz der Agrobakterium tumefaciens-vermittelten Transformation (Standardverfahren) gewonnen. Es wird demgemäß ein Agrobakterium eingesetzt, da es rekombinierte DNA-Moleküle enthält, die Invertaseinhibitor-cDNA in antisense oder sense-Orientierung aufweisen, d.h. in denen die Invertaseinhibitor cDNA 3'-wärts von einem Promotor und funktionell mit diesem verbunden vorliegt. Hierbei werden in einer für viele Pflanzenarten einsetzbaren Ausführungsform Blattstücke transformiert, wobei aus den rekombinanten Zellen auf Antibiotikumhaltigen Medium Primärtransformanten regeneriert werden. Die tatsächlich gewählte Transformationstechnik ist abhängig von der Pflanzenart. Durch Regeneration einer transformierten Pflanzenzelle wird eine transgene Pflanze erhalten.

Der Einfluß der veränderten Expression eines apoplastischen Invertaseinhibitors in Nicotiana tabacum wird im foldenden beschrieben:

Tabak (Nicotiana tabacum) wurde mit der cDNA des apoplastischen Tabak-Invertaseinhibitors (Clon Ntinhl; Greiner et al., a.a.O. 1998) in sense- und antisense-Orientierung transformiert (Agrobakterium tumefaciens-vermittelte Transformation, BinAR-Vektor, CaMV35S-Promotor; Blattscheiben-Transformation gemäß Standardverfahren). Die verwendete cDNA wurde aus einer Zellsuspensionskultur von Tabak gewonnen. Primärtransformanten wurden zunächst über Gewebekultur zu Pflanzen regeneriert und anschließend im Gewächshaus zur Blüte gebracht. Die Samen der Primärtransformanten wurden auf Kanamycin-haltigem Medium ausgesät. Nach steriler Voranzucht wurden die Pflanzen der F1-Generation im Gewächshaus zur Blüte gebracht. Nach Bestäubung wurden in regelmäßigen Zeitabständen die Zellwand-Invertase-Aktivitäten in den Ovarien bestimmt.

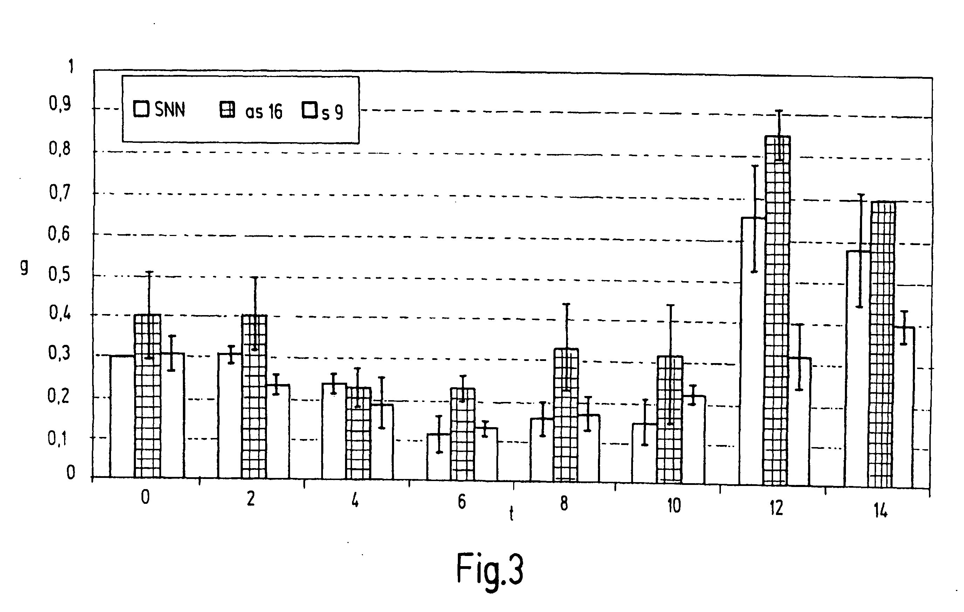

Die Figur 3 zeigt Zellwand-Invertase-Aktivitäten im Ovar von Tabak-Wildtyp (SNN), einer repräsentativen Inhibitor-antisense-Transformante (as16) und einer repäsentativen Inhibitor-sense-Transformante (s9) im Verlauf der frühen Samenentwicklung (0-14 Tage nach Befruchtung). Die Aktivitäten sind in mmol Glucose/g Frischgewicht/min angegeben.

Die Menge an Invertaseinhibitor-Protein wurde über Western Blot-Analyse ermittelt.

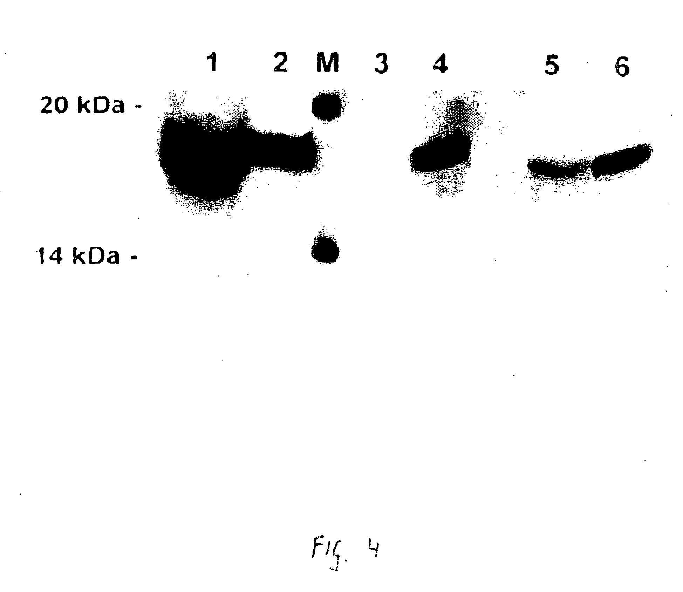

Figur 4 zeigt diesbezüglich den Nachweis der selektiven Reduktion (antisense-Transformante) bzw. Zunahme (sense-Transformante) des Invertaseinhibitor-Polypeptids in Stamina und Ovar, nachgewiesen mit einem gegen den rekombinanten Tabak-Invertaseinhibitor gerichteten Antiserum. (Sense-Transformante (s9): 1, Ovar; 2, Stamina. Antisense-Transformante: 3, Ovar; 4, Stamina. Wildtyp (SNN): 5, Ovar; 6, Stamina). Es wurden über Concanavalin A-Chromatographie gereinigte Proben aufgetragen, in denen nur an Zellwand-Invertase gebundener Inhibitor enthalten ist.

Nach Reifung der Samen wurden deren Trockengewichte bestimmt, sowie die Menge an Speicheröl und Gesamtprotein.

Die Messung der Zellwand-Invertase-Aktivitäten während der frühen Samenentwicklung (Fig.3) zeigt zunächst für den Wildtyp eine ca. 6-fache Steigerung zwischen dem 6. und 12. Tag nach Bestäubung. Dieser Zeitraum entspricht der späten Zellteilungsphase und dem Beginn der Speicherphase. Die Western Blot-Analyse zeigt, daß im Ovar die Menge an Invertaseinhibitor-Polypeptid in antisense-Transformanten stark vermindert, in sense-Transformanten hingegen stark erhöht ist (Fig.4). Im Unterschied hierzu wirkt sich die veränderte Inhibitorexpression in Stamina nur geringfügig aus, vermutlich weil in diesem Gewebe mehrere Inhibitor-Isoformen exprimiert werden.

Die veränderte Aktivität der Zellwand-Invertase während der Samenentwicklung wirkt sich aus auf das Trockengewicht/Samen (Tab.1), den Gehalt an Speicheröl/Samen (Tab.2) und den Gesamtproteingehalt/Samen (Tab.3), nicht jedoch auf die Gesamtzahl an Samen pro Blüte und auch nicht auf die Samengröße.

**Tab.1**

| Trockengewichte pro Samen in Tabak-Wildtyp (SNN), in zwei repräsentativen Inhibitor-sense-Transformanten (s9, s10) und in zwei repräsentativen Inhibitor-antisense-Transformanten (as16, as43). | | |
|---|---|---|
| **Tabak-Linie** | **µg Trockengewicht/Samen** | **Prozent vom Wildtyp (SNN)** |
| WT (SNN) | 64 ± 2 | 100 |
| s9 | 42 ± 2 | 66 |
| s10 | 52 ± 2 | 81 |
| as16 | 71 ± 1 | 110 |
| as43 | 86 ± 4 | 134 |

**Tab.2**

| Samenöl-Gehalte pro Samen in Tabak-Wildtyp (SNN), in zwei repräsentativen Inhibitor-sense-Transformanten (s9, s10) und in zwei repräsentativen Inhibitor-antisense-Transformanten (as16, as43). | | |
|---|---|---|
| **Tabak-Linie** | **µg Gesamtöl/Samen** | **Prozent vom Wildtyp (SNN)** |
| WT (SNN) | 23 | 100 |
| s9 | 10 | 43 |
| s10 | 16 | 69 |
| as16 | 28 | 122 |
| as43 | 39 | 170 |

**Tab.3**

| Gesamtprotein pro Samen in Tabak-Wildtyp (SNN), in zwei repräsentativen Inhibitor-sense-Transformanten (s9, s10) und in zwei repräsentativen Inhibitor-antisense-Transformanten (as16, as43). | | |
|---|---|---|
| **Tabak-Linie** | **µg Gesamtprotein/Samen** | **Prozent vom Wildtyp (SNN)** |
| WT (SNN) | 7.4 | 100 |
| s9 | 5.5 | 74 |
| s10 | 5.7 | 77 |
| as16 | 7.8 | 105 |
| as43 | 9.9 | 134 |

Die starke Erhöhung des Speicherölgehaltes in zwei unabhängigen antisense-Transformanten (+22% bzw. +70%) korreliert mit Zunahmen an Gesamtprotein und Samengewicht, wobei die Zunahme für Speicheröl am stärksten ausgeprägt ist. Bemerkenswerterweise korreliert die Zunahme der Zellwand-Invertase-Aktivität im Ovar während der Samenentwicklung mit der Phase der maximalen Akkumulation von Speicheröl.

Die gesamte vegetative Entwicklungsphase der Inhibitor-antisense- und Inhibitor-sense-Transformanten verläuft ohne sichtbaren Phänotyp, mit Ausnahme des Keimungsvorganges. Hier zeigt sich zwischen der Keimung von Tabak-Wildtyp-Samen und Invertaseinhibitor-antisense-Samen kein Unterschied, wohingegen es bei Samen von Invertaseinhibitor-sense-Transformanten zu einer signifikanten Verzögerung der Keimung kommt.

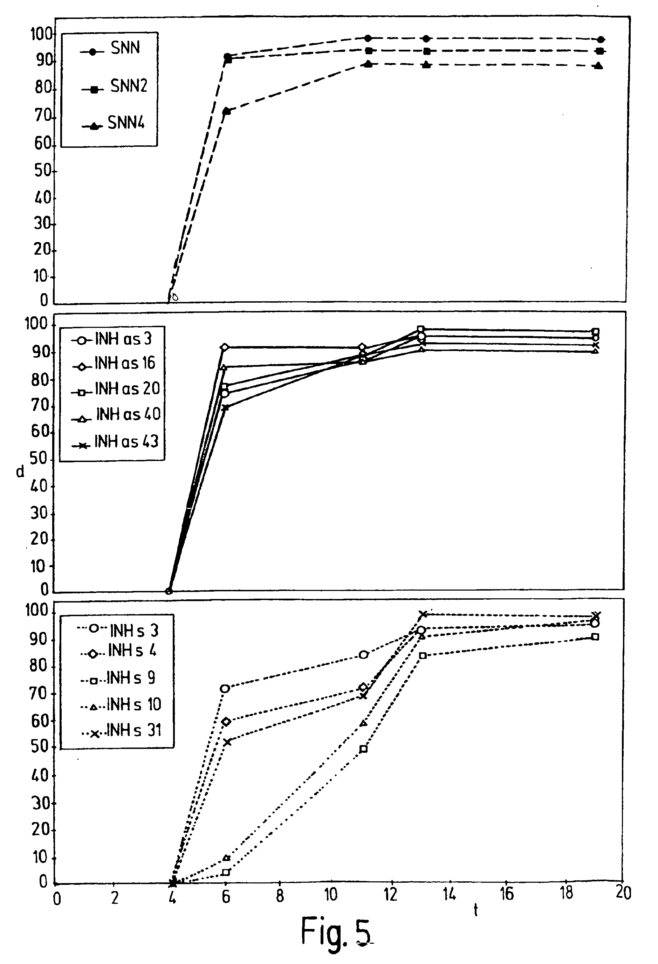

Figur 5 zeigt das Keimungsverhalten von Samen des Tabak-Wildtyps (SNN), von Invertaseinhibitor-antisense-Transformanten (INHas) und von Invertaseinhibitor-sense-Transformanten. Pro Linie wurden unter sterilen Bedingungen jeweils 40 Samen auf LS-Medium (0.5% Saccharose, pH 5.6) ausgesät. Das Hervortreten der Radicula diente als Kriterium für die Keimung.

Ein weiteres Anwendungsbeispiel der vorliegenden Erfindung ist die Einführung eines Invertaseinhibitor-antisense-Konstruktes in Raps (Brassica napus). Raps enthält auf Samenbasis eine dem Tabak vergleichbare Menge an Speicheröl. Das Ergebnis einer Invertaseinhibitor-antisense-Transformation ist eine Erhöhung des Speicherölgehaltes um mindestens 20% , ggf. aber um bis zu 70%.

Eine weitere Anwendung mit dem gleichen Ziel, nämlich der Erhöhung des Speicheröl-Gehaltes, ist die Transformation der Sonnenblume, oder auch die Transformation der Sojabohne, mit einem Invertaseinhibitor-antisense-Konstrukt (Bereitstellung transgener ölspeichernder Pflanzen dieser Spezies).

In einer weiteren Ausführungsform wird durch die Einbringung von Invertaseinhibitor-antisense-Konstrukten in Mais, Reis, Weizen, Hafer, Gerste und Roggen die Menge an Samen-Speicherstärke erhöht. Es können also transgene Pflanzenzellen oder Pflanzen, die Stärke speichern, bereitgestellt werden. Für proteinreiche Samen, z.B. Sojabohne und Erbse, wird in einer weiteren Ausführungsform durch Einführung von Invertaseinhibitor-antisense-Konstrukten die Gesamtmenge an Speicherprotein erhöht.

In einer weiteren Ausführungsform wird durch die Einführung von Invertaseinhibitor-antisense-Konstrukten bzw. durch die daraus resultierende verbesserte Speicherstoffakkumulation die Keimfähigkeit von Samen einer Nutzpflanze erhöht.

Die Bereitstellung transgener Pflanzenzellen oder Pflanzen betrifft vorzugsweise Nutzpflanzen.

## Patentansprüche

1. Verfahren zur Herstellung einer transgenen Pflanze, deren Samen gegenüber Samen von Wildtyppflanzen erhöhte Speicherstoffmengen aufweisen, umfassend
a) die Gewinnung einer Nucleotidsequenz eines Invertaseinhibitorgens einer Pflanze,
b) die Herstellung eines DNA-Konstrukts, enthaltend die mit mindestens einer regulatorischen Einheit funktionell verbundene Nucleotidsequenz des Invertaseinhibitorgens,
c) die Transformation einer Pflanzenzelle einer Pflanze der gleichen Art oder Sorte, aus der die Nucleotidsequenz stammt oder abgeleitet ist, mit dem DNA-Konstrukt und
d) die Kultivierung und Regeneration einer Pflanze.

2. Verfahren nach Anspruch 1, wobei der Invertaseinhibitor ein apoplastischer Invertaseinhibitor ist.

3. Verfahren nach Anspruch 1 oder 2, wobei die Nucleotidsequenz des Invertaseinhibitorgens eine cDNA, eine damit hybridisierende Nucleotidsequenz oder ein Fragment einer der beiden ist.

4. Verfahren nach einem der Ansprüche 1 bis 3, wobei die Nucleotidsequenz des Invertaseinhibitorgens aus einer cDNA-Bank von Blüten mit jungen Samenanlagen einer Pflanze gewonnen wird.

5. Verfahren nach einem der Ansprüche 1 bis 3, wobei die Nucleotidsequenz des Invertaseinhibitorgens durch folgende Schritte gewonnen wird
a) Gewinnung einer Inhibitorproteinfraktion aus der Zellwandprotein-Fraktion von Blüten mit jungen Samenanlagen,
b) Gewinnung von entsprechenden Peptidsequenzen nach Spaltung und Reinigung der entsprechenden Peptide, und
c) Clonierung von zunächst partieller und in der Folge vollängen-cDNA für das Invertaseinhibitorprotein aus einer cDNA-Bank, insbesondere von Blüten mit jungen Samenanlagen in einen Vektor.

6. Verfahren nach einem der Ansprüche 1 bis 5, wobei das DNA-Konstrukt in einem Vektor, insbesondere einem Plasmid oder Virus, vorliegt.

7. Verfahren nach einem der Ansprüche 1 bis 6, wobei die regulatorische Einheit ein Promotor ist.

8. Verfahren nach Anspruch 7, wobei der Promotor ein konstitutiver oder induzierbarer Promotor ist.

9. Verfahren nach Anspruch 8, wobei der Promotor der 35SCaMV Promotor oder der Ubiquitin- oder Zein-Promotor aus Mais ist.

10. Verfahren nach einem der Ansprüche 1 bis 9, wobei die Nucleotidsequenz des Invertaseinhibitorgens sense- oder antisense-Orientierung zu dem Promotor aufweist.

11. Verfahren nach einem der Ansprüche 1 bis 10, wobei das DNA-Konstrukt weitere regulatorische Einheiten, insbesondere ein Transcriptionsterminationssignal, aufweist.

12. Verfahren nach Anspruch 11, wobei das Transcriptionsterminationssignal aus dem NOS-Gen von Agrobacterium tumefaciens stammt.

13. Verfahren nach einem der Ansprüche 1 bis 12, wobei die Pflanzenzelle eine Zelle einer dicotylen oder monocotylen Pflanze ist.

14. verfahren nach Anspruch 13, wobei die Pflanzenzelle eine Zelle von Raps, Sonnenblumen, Erdnuß, Sojabohne, Ölpalme, Reis, Mais, Weizen, Gerste, Hafer, Roggen, Erbse; Calendula officinalis; Coriandrum sativum, Crambe abyssinica, Cuphea ssp., Dimorphotheca pluvialis, Euphorbia lagascae, Euphorbia lathyris, Lesquerella grandiflora, Limnanthes alba, Linum usitatissimum, Lunaria annua, Lunaria biennis, Oenothera ssp., Ricinus communis oder Simmondosia chinensis ist.

15. Verfahren nach einem der Ansprüche 1 bis 14, wobei die Transformation der Pflanzenzelle mittels Agrobakterium tumefaciens, biolistischer Verfahren, mittels elektrisch oder chemisch induzierter DNA-Aufnahme, Elektroporation, Makroinjektion, Mikroinjektion oder PEG-vermittelter Transformation durchgeführt wird.

16. Verwendung einer Nucleotidsequenz eines Invertaseinhibitorgens zur Herstellung von transgenen Pflanzen, deren Samen gegenüber Samen von Wildtyppflanzen erhöhte Speicherstoffmengen aufweisen.

17. Verwendung nach Anspruch 16, wobei die Nucleotidsequenz aus einer cDNA-Bank von Blüten mit jungen Samenanlagen der zu transformierenden Pflanzenart oder -sorte stammt oder davon abgeleitet ist.

18. Verwendung nach Anspruch 16 oder 17, wobei die Nucleotidsequenz des Invertaseinhibitorgens funktionell mit mindestens einer regulatorischen Einheit verbunden ist.

19. Verwendung nach Anspruch 18, wobei die Nucleotidsequenz in antisense-Orientierung zu der mindestens einen regulatorischen Einheit, insbesondere dem Promotor, vorliegt.

## Claims

1. Method of manufacturing a transgenic plant, the seeds of which exhibit increased capacity of storage material in comparison with wild types of plant, comprising
a) obtaining a nucleotide sequence of an invertase inhibitor gene of a plant;
b) manufacturing a DNA structure, containing the nucleotide sequence of the invertase inhibitor gene that is functionally connected with at least a regulatoric unit;
c) transforming a plant cell of a plant of the same type or sort, from which the nucleotide sequence originates or derived from, with the DNA structure; and
d) cultivating and regenerating a plant.

2. Method according to claim 1, wherein the invertase inhibitor is an apoplastic invertase inhibitor.

3. Method according to claim 1 or 2, wherein the nucleotide sequence of the invertase inhibitor gene is a cDNA, thus a hybridising nucleotide sequence or a fragment of one of both.

4. Method according to one of the claims 1 to 3, wherein the nucleotide sequence of the invertase inhibitor gene is derived from a cDNA bank from flowers with young ovules of a plant.

5. Method according to claims 1 to 3, wherein the nucleotide sequence of the invertase inhibitor gene is obtained through the following steps:
a) obtaining an inhibitor protein fraction from the cell wall protein fraction of flowers with young ovules;
b) obtaining the correspondent peptide sequences after splitting and cleaning the respective peptides; and
c) cloning of an initially partial and in succession a full-length cDNA for the invertase inhibitor protein from a cDNA bank, especially from flowers with young ovules in a vector.

6. Method according to claims 1 to 5, wherein the DNA structure exists in a vector, especially a plasmid or virus.

7. Method according to claims 1 to 6, wherein the regulatory unit is a promoter.

8. Method according to claim 7, wherein the promoter is a constitutional or an inducible promoter.

9. Method according to claim 8, wherein the promoter of the 35SCaMV promoter or the ubiquitin promoter or zein promoter is made of maize.

10. Method according to claims 1 to 9, wherein the nucleotide sequence of the invertase inhibitor gene exhibits sense or antisense orientation to the promoter.

11. Method according to claims 1 to 10, wherein the DNA structure exhibits further regulatory units especially a transcription termination signal.

12. Method according to claim 11, wherein the transcription termination signal originates from the NOS gene of the agrobacterium tumefaciens.

13. Method according to claim 1 to 12, wherein the plant cell is a cell of a dicotylen or monocotylen plant.

14. Method according to claim 13, wherein the plant cell is a cell from rape, sunflower, groundnut, soya bean, oil palm, rice, maize, wheat, barley, oats, rye, peas, calendula officinalis, coriandrum sativum, crambe abyssinica, cuphea ssp., dimorphotheca pluvialis, euphorbia lagascae, euphorbia lathyris, lesquerella grandiflora, limnanthes alba, linum usitatissimum, lunaria annua, lunaria biennis, oenothera ssp., ricinus communis or simmondosia chinensis.

15. Method according to one of the claims 1 to 14, wherein the transformation of the plant cell is done by means of agrobacterium tumefaciens, biolistic methods, by means of electrically or chemically induced DNA reception, elecroporation, macroinjection, microinjection or PEG initiated transformation.

16. Application of a nucleotide sequence of an invertase inhibitor gene for manufacturing transgenic plants, by which the seeds in comparison with seeds of wild types of plants exhibit greater quantities of storage material.

17. Application according to claim 16, wherein the nucleotide sequence originates or is derived from a cDNA bank of flowers with young ovules of the type or sort of plants to be transformed.

18. Application according to claim 16 or 17, wherein the nucleotide sequence of the invertase inhibitor gene is functionally connected with at least a regulatory unit.

19. Application according to claim 18, wherein the nucleotide sequence exists in anti-sense orientation to at least a regulatory unit, especially the promoter.

## Revendications

1. Procédé pour la fabrication d'une plante transgénique dont les semences présentent une teneur en substances réservant d'énergie supérieure à celle d'espèces sauvages, comprenant
a) l'obtention d'une séquence nucléotidique d'un gène inhibiteur de l'invertase d'une plante,
b) la fabrication d'un construit ADN, comprenant la séquence nucléotidique du gène inhibiteur de l'invertase fonctionnellement liée à au moins une unité régulatrice,
c) la transformation d'une cellule végétale d'une plante de la même espèce ou variété végétale de laquelle la séquence nucléotidique provient ou de laquelle celle-ci est un dérivé par l'intermédiaire du construit ADN, et
d) la cultivation et la régénération d'une plante.

2. Procédé selon la revendication 1, dans lequel l'inhibiteur d'invertase est un inhibiteur d'invertase apoplastique.

3. Procédé selon la revendication 1 ou 2, dans lequel la séquence nucléotidique du gène inhibiteur de l'invertase est un ADNc, une séquence nucléotidique s'hybridant avec celui-ci ou un fragment de l'une ou de l'autre.

4. Procédé selon l'une des revendications 1 à 3, dans lequel la séquence nucléotidique du gène inhibiteur de l'invertase s'obtient à partir d'une banque ADNc de fleurs avec de jeunes ovules d'une plante.

5. Procédé selon l'une des revendications 1 à 3, dans lequel la séquence nucléotidique du gène inhibiteur de l'invertase s'obtient selon les étapes suivantes :
a) obtention d'une fraction d'un inhibiteur de la protéine à partir de la fraction de protéines de la paroi cellulaire de fleurs avec de jeunes ovules,
b) obtention de séquences de peptides correspondantes après la séparation et la purification des peptides correspondantes, et
c) clonage d'ADNc initialement partiel et ensuite pleine-longueur pour le protéine inhibiteur de l'invertase provenant d'une banque ADNc, notamment de fleurs avec de jeunes ovules dans un vecteur.

6. Procédé selon l'une des revendications 1 à 5, dans lequel le construit ADN est présente dans un vecteur, notamment dans un plasmide ou dans un virus.

7. Procédé selon l'une des revendications 1 à 6, dans lequel l'unité régulatrice est un promoteur.

8. Procédé selon la revendication 7, dans lequel le promoteur est un promoteur constitutif ou inductible.

9. Procédé selon la revendication 8, dans lequel le promoteur est le promoteur 35SCaMV ou le promoteur ubiquitine ou zéine de maïs.

10. Procédé selon l'une des revendications 1 à 9, dans lequel la séquence nucléotidique du gène inhibiteur de l'invertase présente une orientation sens ou antisens par rapport au promoteur.

11. Procédé selon l'une des revendications 1 à 10, dans lequel le construit ADN présente d'autres unités régulatrices, notamment un signal de terminaison de la transcription.

12. Procédé selon la revendication 11, dans lequel signal de terminaison de la transcription provient du gène NOS de l'Agrobacterium tumefaciens.

13. Procédé selon l'une des revendications 1 à 12, dans lequel la cellule végétale est une cellule d'un dicotyle ou d'un monocotyle.

14. Procédé selon la revendication 13, dans lequel la cellule végétale est une cellule de colza, de tournesol, d'arachide, de soya, de palmier oléifère, de riz, de maïs, de blé, d'orge, d'avoine, de seigle, de pois, de Calendula officinalis, de Coriandrum sativum, de Crambe abyssinica, de Cuphea ssp., de Dimorphotheca pluvialis, d'Euphorbia lagascae, d'Euphorbia lathyris, de Lesquerella grandiflora, de Limnanthes alba, de Linum usitatissimum, de Lunaria annua, de Lunaria biennis, d'Oenothera ssp., de Ricinus communis ou de Simmondosia chinensis.

15. Procédé selon l'une des revendications 1 à 14, dans lequel la transformation de la cellule végétale s'effectue par l'intermédiaire d'Agrobacterium tumefaciens, par un procédé biolistique, par incorporation d'ADN électriquement ou chimiquement induite, par électroporation, par macroinjection, par micro-injection ou par l'intermédiaire de PEG.

16. Utilisation d'une séquence nucléotidique d'un gène inhibiteur de l'invertase pour la fabrication de plantes transgéniques dont les semences présentent une teneur en réserves énergétiques supérieure à celle d'espèces sauvages.

17. Utilisation selon la revendication 16, dans laquelle la séquence nucléotidique provient d'une banque ADNc de fleurs avec de jeunes ovules de l'espèce ou de la variété végétale devant être transformée ou en est un dérivé.

18. Utilisation selon la revendication 16 ou 17, dans laquelle la séquence nucléotidique du gène inhibiteur de l'invertase est fonctionnellement liée à au moins une unité régulatrice.

19. Utilisation selon la revendication 18, dans laquelle la séquence nucléotidique est présente dans une orientation antisens par rapport à l'au moins une unité régulatrice, notamment au promoteur.
